# EUROPEAN PATENT SPECIFICATION

(11) **EP 4 458 328 B1**
(45) Date of publication and mention of the grant of the patent: **03.06.2026**
(21) Application number: 22914286.4
(22) Date of filing: 15.12.2022
(51) Int. Cl.: A61F 2/966

(54) **SHEATH CORE ASSEMBLY AND DELIVERY INSTRUMENT**
HÜLLENKERNANORDNUNG UND ABGABEINSTRUMENT
ENSEMBLE ÂME DE GAINE ET INSTRUMENT D'ADMINISTRATION

(30) Priority: 31.12.2021 CN 202111678807; 31.12.2021 CN 202123451901 U; 31.12.2021 CN 202111675505
(43) Date of publication of application: 06.11.2024
(73) Proprietor: Lifetech Scientific (Shenzhen) Co., Ltd., Shenzhen, Guangdong 518063 (CN)
(72) Inventor: LIU, Kui, Shenzhen, Guangdong 518063 (CN); XIAO, Benhao, Shenzhen, Guangdong 518063 (CN)
(74) Representative: Michalski Hüttermann & Partner Patentanwälte mbB
(86) International application number: PCT/CN2022/139209
(87) International publication number: WO 2023/125031

(56) References cited:
- EP-A2- 2 742 879
- WO-A1-2020/011132
- WO-A1-2021/226418
- CN-A- 111 359 077
- CN-U- 211 433 527
- CN-U- 212 679 351
- CN-U- 216 676 051
- US-A1- 2010 268 317
- US-A1- 2013 274 859
- US-A1- 2014 358 215
- US-A1- 2016 270 901
- US-A1- 2019 374 358

## Description

### Cross-Reference to Related Applications

This application claims priorities of Chinese patent application No. 2021116788077 filed with the China National Intellectual Property Administration on December 31, 2021 and entitled "Sheath Core Assembly and Delivery Instrument", Chinese patent application No. 2021234519017 filed with the China National Intellectual Property Administration on December 31, 2021 and entitled "Sheath Core Assembly and Delivery Instrument", and Chinese patent application No. 2021116755054 filed with the China National Intellectual Property Administration on December 31, 2021 and entitled "Sheath Core Assembly and Delivery Instrument'.

### Technical Field

The present invention relates to the technical field of interventional medical instruments, and more particularly to a sheath core assembly and a delivery system.

### Background Art

In recent years, interventional therapy for cardiovascular diseases has become a development trend. With the continuous development of interventional technology, the advantages of using a covered stent in the therapy of aortic aneurysm and arterial dissection have become increasingly prominent. The covered stent is an artificial blood vessel adapted to the size of blood vessels, which is mainly composed of a covered membrane and a metal wave coil supporting the covered membrane. The covered membrane is generally made of a polyester or e-PTFE membrane, and the metal wave coil is mainly woven by a nickel-titanium alloy wire. When the covered stent is delivered to a lesion site for therapy by using a delivery device, the stent is first compressed into a sheath of the delivery device, the blood vessel is punctured, and a track is established by using a guide wire to deliver the delivery device to a designated position of the lesion. Then the stent is released, and the stent is unfolded and attached to the aneurysm vascular wall. The stent covering isolates the blood flow from the lesion site, eliminates the impact of the blood flow on the aneurysm wall at the lesion site, and re-establishes a normal channel of blood circulation. Finally, the guide wire and the delivery device are withdrawn, thus realizing interventional therapy of aneurysm and arterial dissection.

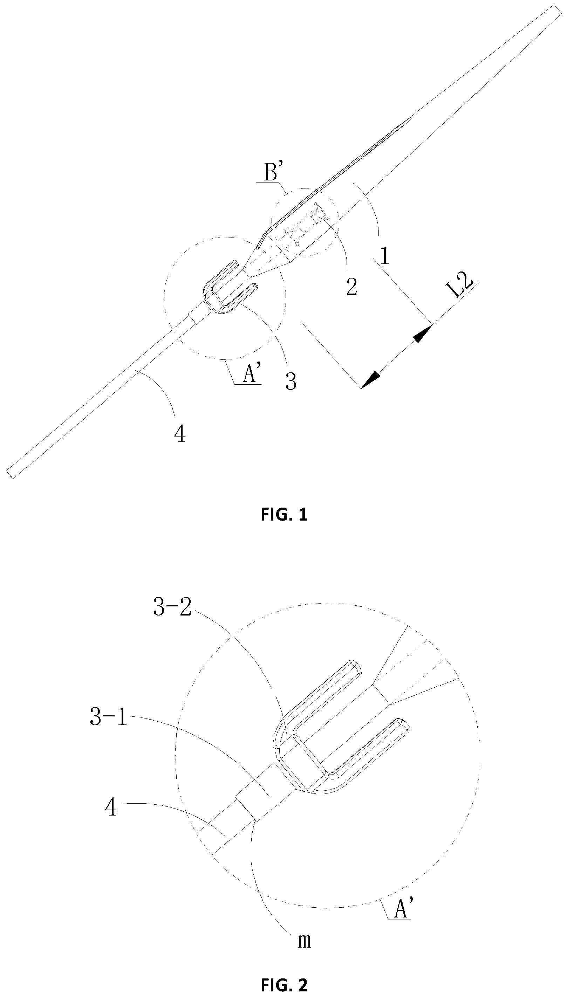

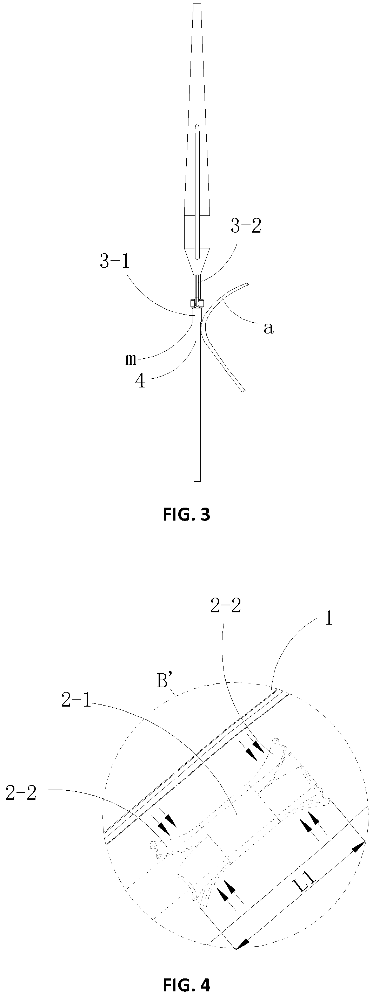

The existing iliac artery bifurcation stent delivery device, as shown in FIG. 1, has a sheath core assembly composed of a tip 1, a bifurcation tube 2, a U-shaped anchor 3, and a sheath core 4. As shown in FIG. 2, the U-shaped anchor 3 is composed of a stent fixing rod 3-2 and a welding zone 3-1. The U-shaped anchor 3 is configured to hook a proximal end of a stent to realize the post-release of the stent. The welding zone 3-1 of the U-shaped anchor 3 is a separate section of stainless steel tube. After the sheath core 4 is welded and fixed to the welding zone 3-1 of the U-shaped anchor 3, a circumferential step m is formed with the sheath core 4 at a distal end of the welding zone 3-1. As shown in FIG. 3, when a sheath core is withdrawn from a curved blood vessel a, the step m of the U-shaped anchor 3 may hook the stent to cause shifting of the stent or may hook a vascular wall and is thus difficult to withdraw.

The structure of the existing bifurcation tube 2 is shown in FIG. 4. The bifurcation tube 2 is configured to connect and fix the tip 1 to the sheath core 4. The bifurcation tube 2 has a welding zone 2-1 and a fixing zone 2-2. The two functional zones are arranged at different positions of the bifurcation tube 2, resulting in a large overall length L1 of the bifurcation tube. Also, since the bifurcation tube 2 is buried in the tip 1, a length L2 of a high-hardness zone of the tip 1 is large, thus reducing the compliance of the tip 1 and affecting the pushing and withdrawing of the punctured tissue of the sheath core 4 in the blood vessel. In addition, when the bifurcation tube 2 is used for injection molding of the tip 1, the fixing zone 2-2 of the bifurcation tube 2 will be deformed or even broken in an arrow direction due to the injection molding pressure (the pressure direction is as indicated by arrows in FIG. 4), which will reduce the connection strength and seriously lead to the connection failure.

The U-shaped anchor 3 is composed of a stent fixing rod 3-2 and a welding zone 3-1. The U-shaped anchor 3 is sleeved to the sheath core 4, and the welding zone 3-1 is fixed to the sheath core 4. In addition, the bifurcation tube 2 is also sleeved to a distal end of the sheath core 4, and the tip wraps the distal end of the sheath core 4, while the sheath core 4 is usually a stainless steel tube, which leads to high hardness and poor compliance of the distal end of the sheath core 4. As shown in FIG. 3, the sheath core cannot bend or recover in time in compliance with the shape of the blood vessel a, and the blood vessel a conflicts with the sheath core to form a resistance zone a1, which will make it difficult to push or withdraw the sheath core.

US 2016/270901A1 provides a stent graft system including a tubular graft component, a bare stent component, an infrarenal stent component at least one suprarenal barb extending distally from at least one suprarenal portion of the bare stent component and at least one infrarenal barb extending distally from at least one infrarenal portion of the bare stent component. A stent graft system can also include at least one barb. WO 2021/226418 A1 dicloses a delivery system for a prosthetic valve device including a first shaft having an outflow capture device mounted on a distal end thereof, a second shaft slidingly disposed over the first shaft and including an inflow capture device mounted on a distal end thereof, and a retractable sheath slidingly disposed over the second shaft to hold the prosthetic valve device in a radially compressed configuration for delivery to a body lumen. The inflow capture device is configured for releasable engagement through openings in a plurality of first attachment members on the device. The outflow capture device is configured for releasable engagement through openings in a plurality of second attachment members on the device.

### Summary of the Invention

Based on the above-mentioned problems, the present invention provides a sheath core assembly and a delivery system, aiming at solving at least one of the above-mentioned technical problems. The invention is defined in the appended set of claims.

To achieve the above object, the present invention adopts a technical solution as follows.

The present invention provides a sheath core assembly, including a sheath core and a U-shaped anchor. The U-shaped anchor includes an anchor body and a stent fixing rod. The anchor body is sleeved to the sheath core and relatively fixed to the sheath core. The anchor body includes a distal face and a proximal face which are opposite to each other, and an outer peripheral face surrounded between the distal face and the proximal face. The distal face is larger than the proximal face, and edge profiles of the distal face and the proximal face are arc-shaped. The outer peripheral face is an arc face having a smooth transition from the distal face to the proximal face. A proximal end of the stent fixing rod is fixedly connected to the distal face of the anchor body. The sheath core assembly further includes a tip wrapping a distal end of the sheath core and a connector sleeved to a distal end of the sheath core and configured to connect the sheath core to the tip. A groove is recessed in an outer peripheral surface of the connector, and the connector is relatively fixed to the sheath core and the tip through the groove.

In one embodiment, the distal face and the proximal face have the same shape.

In one embodiment, both the distal face and the proximal face of the anchor body are round, whereby the anchor body is approximately conical.

In one embodiment, an accommodating groove penetrating the distal face is recessed in the outer peripheral face near a distal end of the anchor body, and the accommodating groove is provided near the stent fixing rod, whereby a connecting arm of a stent hooked on the stent fixing rod is placed in the accommodating groove.

In one embodiment, both sides of the stent fixing rod are provided with the accommodating grooves, whereby the connecting arms connected to both sides of a hooking portion of the stent are accommodated in the accommodating grooves on both sides of the stent fixing rod respectively.

In one embodiment, both the distal face and the proximal face of the anchor body are elliptic, whereby the anchor body is approximately elliptically conical.

In one embodiment, there are two stent fixing rods, which are opposite to each other in a radial direction of the distal face.

In one embodiment, a welding groove is recessed in the outer peripheral face near the proximal end of the anchor body.

In one embodiment, multiple welding grooves are provided at intervals circumferentially along the outer peripheral face of the anchor body.

In one embodiment, the anchor body is axially provided with two symmetrical pulling wire holes, and the pulling wire holes are opposite to exhaust grooves on the tip in the sheath core assembly.

In one embodiment, the connector has a polygonal radial cross-section, whereby an outer peripheral face of the connector includes multiple surfaces, and the groove is recessed in the outer peripheral surface of the connector.

In one embodiment, the connector has a quadrilateral radial cross-section, whereby the outer peripheral face of the connector includes four surfaces, and the groove includes a first groove provided on the surface and penetrating a distal face and a proximal face of the connector and/or includes a second groove recessed circumferentially along the outer peripheral face of the connector.

In one embodiment, the groove includes a third groove recessed circumferentially along the outer peripheral face of the connector, blocking portions are formed at a proximal side and a distal side of the third groove respectively and have quadrilateral radial cross-sections, and two opposite sides of the quadrilateral are straight lines while the other two opposite sides are arc lines, whereby two opposite faces of the blocking portion are arc faces, and the arc faces of the two blocking portions at the proximal side and the distal side are interlaced in azimuth.

In one embodiment, the sheath core includes a first tube body and a second tube body sleeved in the first tube body, a distal end of the second tube body extends out of a distal face of the first tube body, and the compliance of the second tube body is superior to that of the first tube body.

In one embodiment, the first tube body is a cylindrical tube, and the second tube body is a helical tube.

In one embodiment, the first tube body includes a first section sleeved outside the second tube body and a second section connected to a distal end of the first section, and the first section is provided with a notch.

In one embodiment, multiple notches are provided at intervals along an axial direction of the first section.

In one embodiment, the adjacent notches on the first section are provided at equal intervals from a proximal end of the first section to the distal end thereof.

In one embodiment, the adjacent notches on the first section are provided at gradually reducing intervals from a proximal end of the first section to the distal end thereof.

In one embodiment, the notch is a helical notch which extends helically along an axial direction of the first section.

In one embodiment, the adjacent helical notches on the first section are provided at gradually reducing intervals from a proximal end of the first section to the distal end thereof.

In one embodiment, a length between the nearest and farthest notches on the first section is 5-200 mm.

The present invention adopts another technical solution.

The present invention also provides a delivery instrument, including the sheath core assembly as described above.

The present invention adopts yet another technical solution.

The present invention provides a sheath core assembly, including a sheath core and a connector sleeved to a distal end of the sheath core and configured to connect the sheath core to the tip. A groove is recessed in an outer peripheral surface of the connector, and the connector is relatively fixed to the sheath core and the tip through the groove.

In one embodiment, the connector has a polygonal radial cross-section, whereby an outer peripheral face of the connector includes multiple surfaces, and the groove is recessed in the outer peripheral surface of the connector.

In one embodiment, the connector has a quadrilateral radial cross-section, whereby the outer peripheral face of the connector includes four surfaces, and the groove includes a first groove provided on the surface and penetrating a distal face and a proximal face of the connector and/or includes a second groove recessed circumferentially along the outer peripheral face of the connector.

In one embodiment, the groove includes a third groove recessed circumferentially along the outer peripheral face of the connector, blocking portions are formed at a proximal side and a distal side of the third groove respectively and have quadrilateral radial cross-sections, and two opposite sides of the quadrilateral are straight lines while the other two opposite sides are arc lines, whereby two opposite faces of the blocking portion are arc faces, and the arc faces of the two blocking portions at the proximal side and the distal side are interlaced in azimuth.

In one embodiment, the sheath core includes a first tube body and a second tube body sleeved in the first tube body, a distal end of the second tube body extends out of a distal face of the first tube body, and the compliance of the second tube body is superior to that of the first tube body.

In one embodiment, the first tube body is a cylindrical tube, and the second tube body is a helical tube.

In one embodiment, the first tube body includes a first section sleeved outside the second tube body and a second section connected to a distal end of the first section, and the first section is provided with a notch.

In one embodiment, multiple notches are provided at intervals along an axial direction of the first section.

In one embodiment, the adjacent notches on the first section are provided at equal intervals from a proximal end of the first section to the distal end thereof.

In one embodiment, the adjacent notches on the first section are provided at gradually reducing intervals from a proximal end of the first section to the distal end thereof.

In one embodiment, the notch is a helical notch which extends helically along an axial direction of the first section.

In one embodiment, the adjacent helical notches on the first section are provided at gradually reducing intervals from a proximal end of the first section to the distal end thereof.

In one embodiment, a length between the nearest and farthest notches on the first section is 5-200 mm.

The present invention adopts another technical solution.

The present invention also provides a delivery instrument, including the sheath core assembly as described above.

The present invention adopts yet another technical solution.

The present invention provides a sheath core assembly, including a sheath core. The sheath core includes a first tube body and a second tube body sleeved in the first tube body. A distal end of the second tube body extends out of a distal face of the first tube body. The compliance of the second tube body is superior to that of the first tube body.

In one embodiment, the first tube body is a cylindrical tube, and the second tube body is a helical tube.

In one embodiment, the first tube body includes a first section sleeved outside the second tube body and a second section connected to a distal end of the first section, and the first section is provided with a notch.

In one embodiment, multiple notches are provided at intervals along an axial direction of the first section.

In one embodiment, the adjacent notches on the first section are provided at equal intervals from a proximal end of the first section to the distal end thereof.

In one embodiment, the adjacent notches on the first section are provided at gradually reducing intervals from a proximal end of the first section to the distal end thereof.

In one embodiment, the notch is a helical notch which extends helically along an axial direction of the first section.

In one embodiment, the adjacent helical notches on the first section are provided at equal or gradually reducing intervals from a proximal end of the first section to the distal end thereof.

In one embodiment, a length between the nearest and farthest notches on the first section is 5-200 mm.

The present invention adopts another technical solution.

The present invention also provides a delivery instrument, including the sheath core assembly as described above.

The present invention provides a sheath core assembly and a delivery instrument including the sheath core assembly. An outer periphery of an anchor body is arranged as an arc-shaped transition face, and a welding groove is recessed in the arc-shaped transition face, whereby a distal end of the anchor body is stepless, thus solving the problem of difficulty in pushing and withdrawing due to steps in a welding zone, and greatly improving the smoothness of withdrawal of a delivery device in blood vessels, especially curved blood vessels. **In** addition, built-in welding spots avoid damage on the blood vessels by surface unevenness caused by welding, thus further improving the smoothness.

The present invention provides a sheath core assembly and a delivery instrument including the sheath core assembly. By designing a welding zone and a circumferential fixing zone of a connector in the same groove, an overall length of the connector is obviously smaller than the length of the existing bifurcation tube. By reducing the overall length, after the connector is buried in a tip, the length of a high-hardness zone of the tip is smaller, and the compliance is better. At the same time, the overall structure of the connector is more stable, and the connector will not be deformed during injection molding of the tip and is more reliably connected to the tip, thus solving the problems of difficulty in pushing and withdrawing and the fracture failure caused by the existing bifurcation tube.

The present invention provides a sheath core assembly and a delivery instrument including the sheath core assembly. A sheath core 10 is designed by combining a helical tube and a stainless steel tube. The stainless steel tube wraps an outer wall of the helical tube. The sheath core adopts the structure that the helical tube is located at a distal end while the stainless steel tube is located at a proximal end. A notch is provided at a distal end of the stainless steel tube, and the distal end is a closed section for being welded and fixed to the helical tube and sealing the notch. The notch achieves a flexible even transition between the helical tube and the stainless steel tube, ensures the flexibility of the distal end of the sheath core 10 and the rigidity of the proximal end, ensures that a main body of the sheath core 10 has sufficient pushing property, and solves the problem of difficulty in pushing and withdrawing caused by the poor compliance of the distal end of the existing sheath core.

### Brief Description of the Drawings

FIG. 1 is a schematic partial view of an existing sheath core assembly;
FIG. 2 is a schematic enlarged view of part A' in FIG. 1;
FIG. 3 is a schematic view of hooking a blood vessel wall by a step of a U-shaped anchor of an existing sheath core assembly;
FIG. 4 is a schematic enlarged view of part B' in FIG. 1;
FIG. 5 is a schematic view of an existing sheath core assembly with a tip hidden;
FIG. 6 is a schematic view of a sheath core of an existing sheath core assembly in a blood vessel;
FIG. 7 is a schematic view depicting a structure of a sheath core assembly according to the present invention;
FIG. 8 is a schematic enlarged view of part A in FIG. 7;
FIG. 9 is a schematic view depicting a structure of another U-shaped anchor in a sheath core assembly according to the present invention;
FIG. 10 is a schematic view of a sheath core assembly with a tip hidden according to the present invention;
FIG. 11 is a schematic enlarged view of part B in FIG. 10;
FIG. 12 is a schematic view depicting a structure of another connector in a sheath core assembly according to the present invention;
FIG. 13 is a schematic view depicting a structure of yet another connector in a sheath core assembly according to the present invention;
FIG. 14 is a schematic view depicting a structure of yet another connector in a sheath core assembly according to the present invention;
FIG. 15 is a schematic enlarged view of part C in FIG. 10;
FIG. 16 is a schematic view 1 depicting a partial structure of a sheath core in a sheath core assembly according to the present invention; and
FIG. 17 is a schematic view 2 depicting a partial structure of a sheath core in a sheath core assembly according to the present invention.

### Detailed Description of the Invention

In order that the objects, technical solutions, and advantages of the present invention will become more apparent, exemplary implementations of the present disclosure will be described in detail below with reference to the accompanying drawings. While the drawings show exemplary implementations of the present disclosure, it should be understood that the present disclosure may be embodied in various forms and should not be limited by the implementations set forth herein. Rather, these implementations are provided so that the present disclosure will be thoroughly understood, and the scope of the present disclosure will be fully conveyed to those skilled in the art.

It should be understood that the terms used herein are for the purpose of describing particular example implementations only and are not intended to be limiting. As used herein, the singular forms "a/an", "one", and "the" are intended to include the plural forms as well, unless the context clearly indicates otherwise. The terms "comprising", "including", "containing", and "having" are inclusive and therefore specify the presence of stated features, steps, operations, elements, and/or components, but do not preclude the presence or addition of one or more other features, steps, operations, elements, components, and/or combinations thereof. The method steps, processes, and operations described herein are not to be construed as necessarily requiring to be performed in the particular order described or illustrated, unless an order of performance is explicitly stated. It should also be understood that additional or alternative steps may be used.

Although the terms first, second, third, and the like may be used herein to describe various elements, components, regions, layers, and/or sections, these elements, components, regions, layers, and/or sections should not be limited by these terms. These terms may be used only to distinguish one element, component, region, layer, or section from another region, layer, or section. The use of terms such as "first", "second", and other numerical terms herein does not imply a sequence or order unless the context clearly dictates otherwise. Therefore, a first element, component, region, layer, or section discussed below may be referred to as a second element, component, region, layer, or section without departing from the teachings of example implementations.

For ease of description, spatially relative terms, such as "inner", "outer", "inside", "outside", "underneath", "below", "over", and "above", may be used herein to describe the relationship between one element or feature and another element or feature as illustrated in the figures. Such spatially relative terms are intended to encompass different orientations of a device in use or operation in addition to the orientations depicted in the figures. For example, if the device in the figures is turned over, elements described as "underneath" or "below" other elements or features would then be oriented "over" or "above" the other elements or features. Therefore, the example term "below" may include orientations: above and below. The device may be otherwise oriented (rotated by 90 degrees or in other directions) and is interpreted accordingly by spatially relative descriptors used herein.

It should be additionally noted that in the field of interventional medical instruments, an end of a medical instrument implanted in a human or animal body or a delivery system for delivering the medical instrument, which is closer to an operator, is generally referred to as a "proximal end", an end which is further away from the operator is referred to as a "distal end", and the "proximal end" and "distal end" of any component of the medical instrument or the delivery system are defined in accordance with this principle. An "axial direction" generally refers to a lengthwise direction of the medical instrument when being delivered, and a "radial direction" generally refers to a direction of the medical instrument perpendicular to the "axial direction" thereof, and the "axial direction" and "radial direction" of any component of the medical instrument are defined in accordance with this principle.

### Embodiment 1

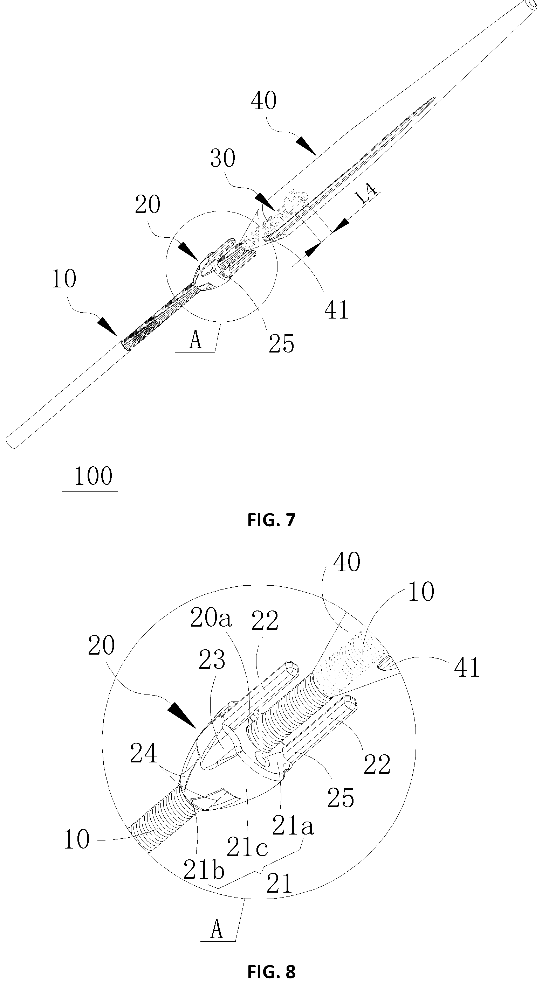

Referring to FIG. 7, in view of the problem that a circumferential step of a U-shaped anchor in an existing delivery device hooks a stent to cause shifting of the stent or hooks a vascular wall and is thus difficult to withdraw, the present invention exemplarily provides a sheath core assembly 100 such that a delivery device including the sheath core assembly 100 of this embodiment may be smoothly pushed and withdrawn in a blood vessel.

Specifically, referring to FIG. 7, the sheath core assembly 100 of this embodiment includes a sheath core 10 and a U-shaped anchor 20 sleeved to the sheath core 10 and relatively fixed to the sheath core 10. In addition, the sheath core assembly 100 may also include a tip 40 wrapping a distal end of the sheath core 10. The structure of the sheath core 10 in this embodiment may refer to the prior art. For example, the sheath core may be a stainless steel tube. Preferably, a novel sheath core structure may also be provided. A detailed implementation of the novel sheath core structure may refer to the following Embodiment 3. The structure of the U-shaped anchor 20 in the sheath core assembly 100 is mainly described in detail in this embodiment.

As shown in FIG. 7 and FIG. 8, the U-shaped anchor 20 of this embodiment is different from the prior art in that the U-shaped anchor includes an anchor body 21 and a stent fixing rod 22. The anchor body 21 is axially provided with a via 20a. The anchor body 21 is sleeved to the sheath core 10 and relatively fixed to the sheath core 10 through the via 20a. The anchor body 21 includes a distal face 21a and a proximal face 21b which are opposite to each other, and an outer peripheral face 21c surrounded between the distal face 21a and the proximal face 21b. The distal face 21a is larger than the proximal face 21b, and edge profiles of the distal face 21a and the proximal face 21b are arc-shaped, whereby the outer peripheral face 21c is an arc face having a smooth transition from the distal face 21a to the proximal face 21b. A proximal end of the anchor body 21 of this embodiment is stepless. Preferably, the distal face 21a and the proximal face 21b have the same shape in order to ensure the smooth transition of the outer peripheral face. With this arrangement, the outer peripheral face 21c formed by the distal face 21a and the proximal face 21b is free of edges and corners, thus ensuring smoothness of pushing and withdrawing.

A proximal end of the stent fixing rod 22 is fixedly connected to the distal face 21a of the anchor body 21. The stent fixing rod 22 is configured to hook a bare wave coil of a stent. After a wire is fixed to the U-shaped anchor 20, the stent may be brought into a sheath together to complete the assembly. In addition, the U-shaped anchor 20 also plays the role of the post-release of the stent.

Referring to FIG. 8, as an implementation of the anchor body 21 of this embodiment, both the distal face 21a and the proximal face 21b of the anchor body 21 are round, whereby the anchor body 21 is approximately conical, which means that the proximal face 21b of the anchor body 21 is as close as possible to an outer periphery of the sheath core 10 to which the anchor body is relatively fixed, whereby the proximal end of the anchor body 21 is stepless. Preferably, an edge of a joint between the distal face 21a and the outer peripheral face 21c of the anchor body 21 is rounded to improve smoothness.

With continued reference to FIG. 8, when the anchor body 21 is approximately conical, an accommodating groove 23 penetrating the distal face 21a is recessed in the outer peripheral face 21c near a distal end of the anchor body 21 preferably. The accommodating groove 23 is provided near the stent fixing rod 22, whereby a connecting arm of a stent hooked on the stent fixing rod 22 is placed in the accommodating groove 23. Since the space between an outer wall of the U-shaped anchor 20 and an inner wall of a sheath is limited, the accommodating groove 23 serves to enlarge the accommodating space between the sheath and the U-shaped anchor 20, whereby the bare wave of the stent can be accommodated by the sheath, thus avoiding the phenomenon that the bare wave of the stent is protruded after being hung on the stent fixing rod 22, the stent is difficult to be sheathed into the sheath or the sheath is damaged when the stent is sheathed. In addition, the accommodating groove 23 accommodates the bare wave of the stent therein to fix the bare wave of the stent, so as to prevent the bare wave from shaking and damaging the sheath when the stent is sheathed. Preferably, since the stent fixing rod 22 is fixedly connected to the distal face 21a of the anchor body 21, after the stent is hooked on the stent fixing rod 22, the bare wave of the stent, which is the connecting arm of the stent, is inclined relative to the center thereof. In view of this, the depth of the accommodating groove 23 of this embodiment may be gradually increased from a proximal end to a distal end thereof, whereby the distal end of the accommodating groove 23 is inclined and approached towards the center thereof, so as to better comply with the shape of a compressed bare wave coil of the stent, whereby the connecting arm of the stent may be completely placed in the accommodating groove 23, thus preventing the stent from being worn by the friction between the anchor body 21 and the connecting arm of the stent, and facilitating separation during the release process.

Preferably, as shown in FIG. 8, since a single wave of the stent is usually V-shaped, there are actually two connecting arms. Therefore, both sides of the stent fixing rod 22 are provided with the accommodating grooves 23, whereby the connecting arms connected to both sides of a hooking portion of the stent are accommodated in the accommodating grooves 23 on both sides of the stent fixing rod 22 respectively. Further, there are two stent fixing rods 22, and the two stent fixing rods 22 are opposite to each other in a radial direction of the distal face 21a. It will be understood that when there are two stent fixing rods 22, both sides of each stent fixing rod 22 are provided with the accommodating grooves 23. It should be noted that, in other embodiments, the number of the stent fixing rods 22 may be set according to actual needs, and the number of the accommodating grooves 23 at the sides of the stent fixing rods 22 and the provision manner may be improved according to needs, which are not limited thereto.

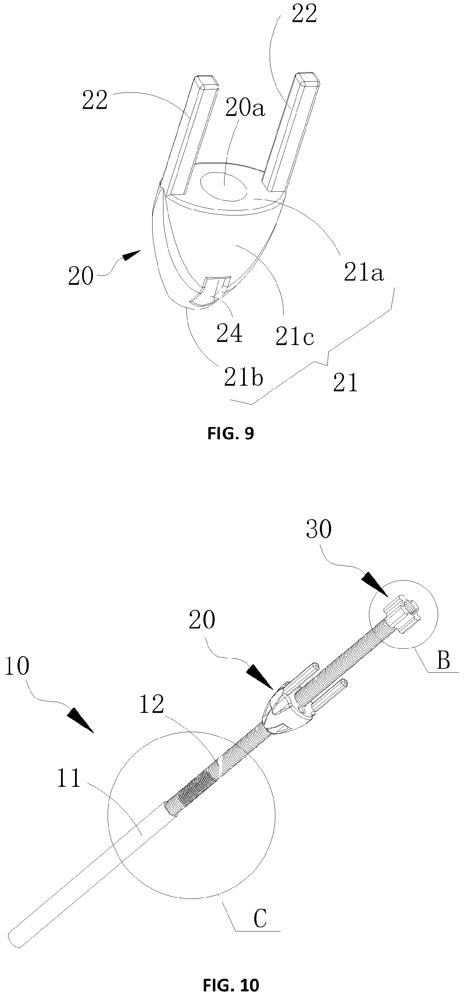

Referring to FIG. 9, as another implementation of the anchor body 21 of this embodiment, the anchor body 21 is axially provided with a via 20a. The anchor body 21 is sleeved to the sheath core 10 and relatively fixed to the sheath core 10 through the via 20a. Both the distal face 21a and the proximal face 21b of the anchor body 21 are elliptic, whereby the anchor body 21 is approximately elliptically conical, which means that the proximal face 21b of the anchor body 21 is as close as possible to an outer periphery of the sheath core 10 to which the anchor body is relatively fixed, whereby the proximal end of the anchor body 21 is stepless. The approximately elliptically conical anchor body 21 is provided such that there is a large gap between the outer periphery of the anchor body 21 and the sheath during the mounting process, and this gap allows passage of the connecting arm of the stent hooked on the stent fixing rod 22 without providing an additional accommodating groove 23. The structure is simplified, and the difficulty and cost of machining are greatly reduced. Preferably, an edge of a joint between the distal face 21a and the outer peripheral face 21c of the anchor body 21 is rounded to improve smoothness.

Further, as shown in FIG. 9, in order to reduce the volume of the U-shaped anchor 20 as much as possible and improve the smoothness of pushing besides realizing basic functions of the U-shaped anchor 20, when the anchor body 21 is arranged in an approximately elliptically conical shape, two stent fixing rods 22 are respectively fixed on edges of two long axes of the distal face of the anchor body 21, and after the via 20a is provided in the axial direction of the anchor body 21, larger connection positions are reserved on both sides of the long axes, whereby the U-shaped anchor 20 may be smaller and may be withdrawn more smoothly.

As shown in FIG. 1 and FIG. 2, the U-shaped anchor 3 is a machined metal member, and the sheath core 4 is a stainless steel metal member in the prior art. In order to realize the relative fixation between the U-shaped anchor 3 and the sheath core 4, an additional welding zone 3-1 is required in the prior art to realize the welding fixation between the U-shaped anchor and the sheath core. To this end, referring to FIG. 8 and FIG. 9, in this embodiment, a welding groove 24 is recessed in the outer peripheral face 21c near the proximal end of the anchor body 21. After the anchor body 21 is sleeved to the sheath core 10 through the via 20a, the anchor body 21 and the sheath core 10 are welded and fixed by the welding groove 24. In this way, an additional welding zone is not required, thus avoiding generation of a step and length increase caused by the welding zone. At the same time, the welding groove 24 of this embodiment is provided such that welding spots are built-in and not on the surface of the U-shaped anchor 20, thus avoiding damage on the blood vessels by surface unevenness caused by welding.

Exemplarily, multiple welding grooves 24 are provided at intervals circumferentially along the arc-shaped outer peripheral face 21c of the anchor body 21. In this embodiment, multiple means two or more. In order to ensure the connection effect, the welding grooves 24 are provided at intervals circumferentially along the outer peripheral face 21c of the anchor body 21. Further, four welding grooves 24 are provided at equal intervals circumferentially.

As shown in FIG. 7 and FIG. 8, in other embodiments, the anchor body 21 is further axially provided with two symmetrical pulling wire holes 25. The pulling wire holes 25 are configured for passage of pulling wires pulling the stent into the sheath. Further, the pulling wire holes 25 are opposite to exhaust grooves 41 on the tip 40 in the sheath core assembly 100. The pulling wire holes 25 and the exhaust grooves 41 of the tip 40 are located on the same side, whereby the pulling wires pass through the tip 40 straightly.

In the sheath core assembly 100 of this embodiment, an outer periphery of an anchor body is arranged as an arc-shaped transition face, and a welding groove is recessed in the arc-shaped transition face, whereby a distal end of the anchor body is stepless, thus solving the problem of difficulty in pushing and withdrawing due to steps in a welding zone, and greatly improving the smoothness of withdrawal of a delivery device in blood vessels, especially curved blood vessels. In addition, built-in welding spots avoid damage on the blood vessels by surface unevenness caused by welding, thus further improving the smoothness.

Further, referring to FIG. 1 and FIG. 4, as mentioned in the Background Art, the sheath core assembly of the existing delivery device further includes a bifurcation tube 2 for connecting and fixing the tip 1 to the stainless steel tube 4. The structure of the existing bifurcation tube 2 is shown in FIG. 4. The bifurcation tube 2 has a welding zone 2-1 and a fixing zone 2-2. The two functional zones are arranged at different positions of the bifurcation tube 2, resulting in a large overall length L1 of the bifurcation tube. Also, since the bifurcation tube 2 is buried in the tip 1, a length L2 of a high-hardness zone of the tip 1 is large, thus reducing the compliance of the tip 1 and affecting the pushing and withdrawing of the punctured tissue of the sheath core 4 in the blood vessel. In addition, when the bifurcation tube 2 is used for injection molding of the tip 1, the fixing zone 2-2 of the bifurcation tube 2 will be deformed or even broken in an arrow direction due to the injection molding pressure (the pressure direction is as indicated by arrows in FIG. 4), which will reduce the connection strength and seriously lead to the connection failure.

In view of this, as an optimized scheme, on the basis of solving the problem of shifting of the stent or difficulty in withdrawing the stent hooked on the vascular wall caused by the step, this embodiment further solves the problem of fracture failure.

In an optimized scheme, the sheath core assembly of this embodiment further includes a connector 30 sleeved to a distal end of the sheath core 10 and configured to connect the sheath core 10 to the tip 40 on the basis of the foregoing. In a further optimized embodiment, the sheath core assembly includes a sheath core 10, a U-shaped anchor 20 sleeved to the sheath core 10 and relatively fixed to the sheath core 10, and a connector 30 sleeved to a distal end of the sheath core 10 and configured to connect the sheath core 10 to the tip 40.

Exemplarily, a groove is recessed in an outer peripheral surface of the connector 30, and the connector is relatively fixed to the sheath core and the tip through the groove. With the groove, the thickness of the groove can be reduced, thus realizing the welding between the connector 30 and the sheath core 10 through the groove. In addition, during the injection molding process of the tip 40, glue injection liquid of the tip 40 may enter the groove to realize the connection between the tip 40 and the connector 30, while forming axial and circumferential limits.

Preferably, the connector 30 for connecting the sheath core 10 to the tip 40 is a solid metal member. Exemplarily, the connector is machined from a stainless steel material. The connector 30 is axially provided with an axial hole 31. The connector 30 is sleeved to the distal end of the sheath core 10 and relatively fixed to the sheath core 10 through the axial hole 31. A groove is recessed in an outer peripheral surface of the connector 30, and the connector 30 is relatively fixed to the sheath core 10 and the tip 40 through the groove.

Exemplarily, the connector 30 has a polygonal radial cross-section, whereby an outer peripheral face of the connector 30 includes multiple surfaces, and the groove is recessed in the outer peripheral surface of the connector 30. This arrangement further enhances the connection and improves the axial and circumferential limits. The groove may be provided on one surface, grooves are provided on multiple surfaces, or multiple grooves are provided on one surface, which may be specifically set according to the situation. It should also be noted that the radial cross-section of the connector 30 may be of other shapes, and is not limited to a polygon. For example, the radial cross-section of the connector may be round, and the connector 30 may be a cylindrical body with a groove recessed in a surface of the cylindrical body.

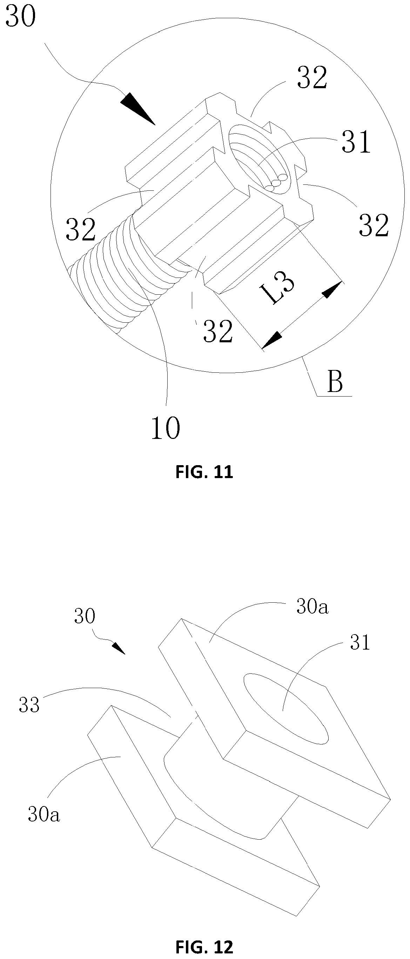

As shown in FIG. 11, as an implementation, the connector 30 has a quadrilateral radial cross-section, whereby the outer peripheral face of the connector 30 includes four surfaces. Preferably, for ease of machining, the connector 30 is a cuboid or a cube, where a circumferential surface of the cuboid or the cube is provided with a groove. Exemplarily, each outer peripheral surface of the cuboid or the cube is provided with a first groove 32 penetrating a distal face and a proximal face of the connector 30, so as to form a strip-shaped groove on each outer peripheral surface. The groove bottom of the strip-shaped groove is welded to the sheath core 10 to achieve relative fixation between the connector 30 and the sheath core 10.

As shown in FIG. 12, as another implementation, the connector 30 has a quadrilateral radial cross-section, whereby the outer peripheral face of the connector 30 includes four surfaces. Preferably, for ease of machining, the connector 30 is a cuboid or a cube, where a circumferential surface of the cuboid or the cube is provided with a second groove 33 not penetrating the distal face and the proximal face of the connector 30. Exemplarily, the second groove 33 is an annular groove surrounding an outer peripheral surface of the cuboid or the cube. The groove bottom of the annular groove is welded to the sheath core 10 to achieve relative fixation between the connector 30 and the sheath core 10.

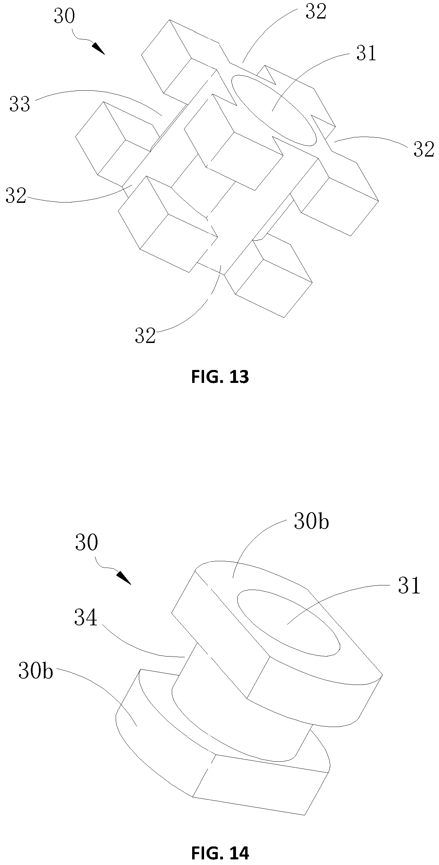

As shown in FIG. 13, as yet another implementation, the connector 30 has a quadrilateral radial cross-section, whereby the outer peripheral face of the connector 30 includes four surfaces. Preferably, for ease of machining, the connector 30 is a cuboid or a cube, where a circumferential surface of the cuboid or the cube is provided with a groove. Exemplarily, each outer peripheral surface of the cuboid or the cube is provided with a first groove 32 penetrating a distal face and a proximal face of the connector 30, so as to form a strip-shaped groove on each outer peripheral surface. At the same time, a circumferential surface of the cuboid or the cube is provided with a second groove 33 not penetrating the distal face and the proximal face of the connector 30. The second groove 33 is provided annularly. The annular groove penetrates through the strip-shaped groove. The groove bottoms of the grooves are welded to the sheath core 10 to achieve relative fixation between the connector 30 and the sheath core 10.

As shown in FIG. 14, as an implementation that the connector 30 has a quadrilateral radial cross-section, a third groove 34 is recessed circumferentially along the outer peripheral face of the connector 30. The third groove 34 does not penetrate the distal face and the proximal face of the connector 30, whereby blocking portions 30b are formed at a proximal side and a distal side of the third groove 34 respectively. The blocking portions 30b have quadrilateral radial cross-sections, and two opposite sides of the quadrilateral are straight lines while the other two opposite sides are arc lines, whereby two opposite faces of the blocking portion 30b are arc faces, and the arc faces of the two blocking portions 30b at the proximal side and the distal side are interlaced in azimuth. The interlaced blocking portions 30b further improve the strength of the circumferential limit and the axial limit.

In this embodiment, the welding zone and the circumferential fixing zone of the connector 30 are designed in the same groove. As shown in FIG. 11 compared to FIG. 4, an overall length L3 of the connector 30 of this embodiment is obviously smaller than a length L4 of the existing bifurcation tube, and the overall length is reduced. As shown in FIG. 7, after being buried in the tip 40, the length L4 of a high-hardness zone of the tip 40 is smaller, and the compliance is better. At the same time, the overall structure of the connector 30 is more stable, and the connector will not be deformed during injection molding of the tip 40 and is more reliably connected to the tip 40. The sheath core assembly 100 of this embodiment not only solves the problem of difficulty in withdrawing caused by the steps in the welding zone, but also solves the problems of difficulty in pushing and withdrawing and fracture failure caused by the existing bifurcation tube.

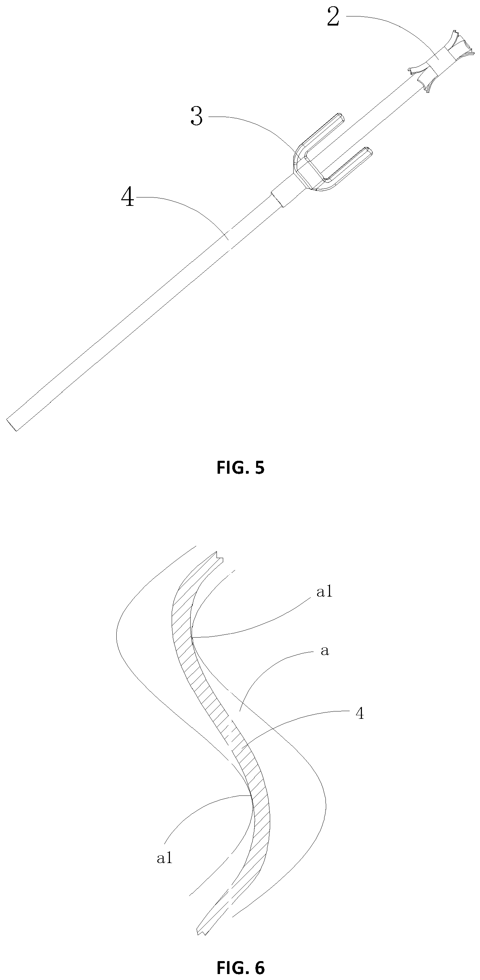

Further, as shown in FIG. 1 and FIG. 6, as mentioned in the Background Art, the existing sheath core assembly includes a sheath core 4. The sheath core 4 is usually a stainless steel tube, which leads to high hardness and poor compliance of a distal end of the sheath core 4. The sheath core cannot bend or recover in time in compliance with the shape of a blood vessel a, and the blood vessel a conflicts with the sheath core 4 to form a resistance zone a1, which will make it difficult to push or withdraw the sheath core.

In view of this, as an optimized scheme, on the basis of solving the problem of shifting of the stent or difficulty in withdrawing the stent hooked on the vascular wall caused by the step, this embodiment further solves the problem of difficulty in pushing and withdrawing caused by high hardness of the distal end of the sheath core.

In an optimized scheme, the sheath core assembly of this embodiment includes a sheath core 10 and a U-shaped anchor 20 sleeved to the sheath core 10 and relatively fixed to the sheath core 10. In a more optimized scheme, the sheath core assembly 100 may further include a tip 40 wrapping the distal end of the sheath core 10. It should be noted that the tip 40 does not constitute a necessary feature of this embodiment to solve the problems presented therein. It should be noted that in addition to the structure of the sheath core 10 and the U-shaped anchor 20 in this embodiment, the structure of other features may refer to the prior art or other embodiments of this application, and the structure of other features is not limited.

Specifically, as shown in FIG. 7, FIG. 10, and FIG. 15, the sheath core 10 of this embodiment includes a first tube body 11 and a second tube body 12 sleeved in the first tube body 11. A distal end of the second tube body 12 extends out of a distal face of the first tube body 11, and the compliance of the second tube body 12 is superior to that of the first tube body 11. The U-shaped anchor 20 is sleeved to the second tube body 12 and relatively fixed to the second tube body 12. In this embodiment, the second tube body 12 having better compliance is sleeved to the first tube body 11 and extends out of the distal end of the first tube body 11, thereby improving the hardness of the distal end of the sheath core 10, improving the compliance of the distal end of the sheath core 10, and solving the problem of difficulty in pushing and withdrawing caused by high hardness of the distal end of the sheath core 10.

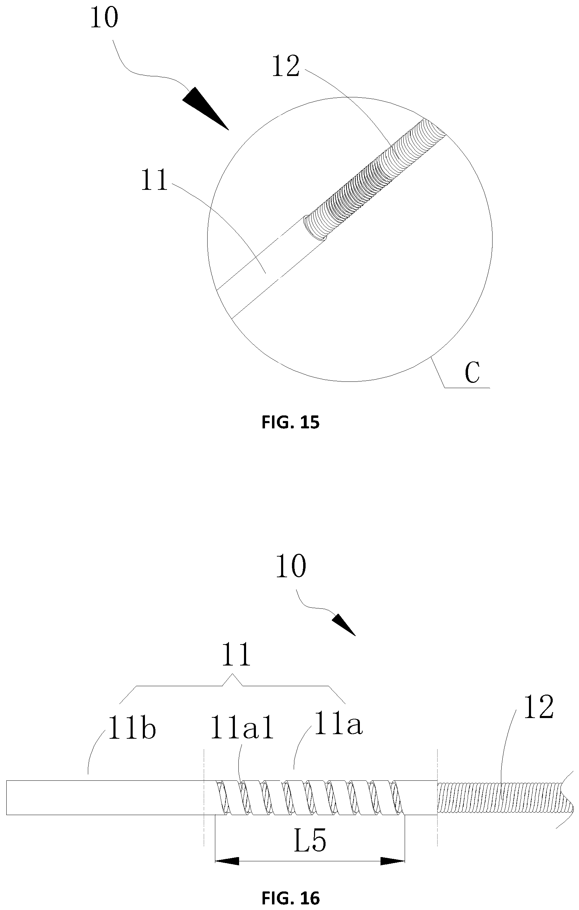

Exemplarily, referring to FIG. 15, the first tube body 11 is a cylindrical tube, preferably a stainless steel tube. The second tube body 12 is a helical tube, having a helical structure similar to a spring. The stainless steel tube wraps an outer wall of the helical tube, and a distal end of the helical tube extends out of a distal end of the stainless steel tube. The compliance of the helical tube is superior to that of the stainless steel tube, and the rigidity of the distal end is ensured while the flexibility of the proximal end of the sheath core 10 is ensured, and a main body of the sheath core 10 has sufficient pushing property.

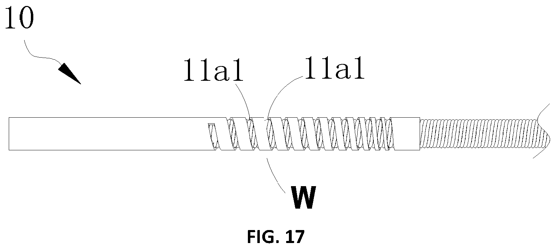

There is a gap between the helical tube and the stainless steel tube because of the sleeve connection between the helical tube and the stainless steel tube, and there is usually a final cleaning process in the manufacturing process, in which cleaning liquid will enter the gap and flush the gap at the same time. In view of this, as shown in FIG. 16 and FIG. 17, the first tube body 11 of this embodiment includes a first section 11a sleeved outside the second tube body 12 and a second section 11b connected to a distal end of the first section 11a. The first section 11a is provided with a notch 11a1. The sleeved stainless steel tube and helical tube can be fully flushed through the notch 11a1, and flushing liquid can easily flow out of the notch after the cleaning process, thus avoiding the cleaning liquid remaining in the gap. At the same time, the hardness of a sleeving section between the first tube body 11 and the second tube body 12 is transitioned to avoid high local hardness, whereby the compliance of the sheath core 10 is better. It should be noted that the distal end of the first section 11a is closed so that the end has a closed section by which the distal ends of the first tube body 11 and the second tube body 12 can be welded.

Preferably, multiple notches 11a1 are provided at intervals along an axial direction of the first section 11a, and the entire sleeving section can be fully cleaned by the multiple notches 11a1 in the axial direction while the hardness of the entire section is transitioned. As an implementation, the adjacent notches 11a1 on the first section 11a are provided at equal intervals from a proximal end of the first section 11a to the distal end thereof. As another implementation, the adjacent notches 11a1 on the first section 11a are provided at gradually reducing intervals from a proximal end of the first section 11a to the distal end thereof. The distance between the proximal and distal notches 11a1 is gradually reduced, whereby the adjacent notches 11a1 are gradually increased from the proximal end to the distal end, and the hardness transition from the proximal end to the distal end is better, and the flexibility towards the distal end is better.

With continued reference to FIG. 16 and FIG. 17, in other embodiments, the notch 11a1 is a helical notch which extends helically along an axial direction of the first section 11a in order to facilitate the cleaning of the gap within the entire sleeving section at the same time as machining. Preferably, as an implementation, the adjacent notches 11a1 on the first section 11a are provided at equal intervals W from a proximal end of the first section 11a to the distal end thereof. As another implementation, the adjacent notches 11a1 on the first section 11a are provided at gradually reducing intervals W from a proximal end of the first section 11a to the distal end thereof, whereby the adjacent notches 11a1 are gradually increased from the proximal end to the distal end, and the transition from the proximal end to the distal end is better, and the flexibility towards the distal end is better.

In the sleeving process, preferably, as shown in FIG. 16, a length L5 between the distal and the proximal notches 11a1 on the first section 11a of this embodiment is 5-200 mm. This length orientation ensures as much as possible the compliance of the transition section and the rigidity of other sections of the sheath core 10, whereby the main body of the sheath core 10 has sufficient pushing property.

In this embodiment, the sheath core 10 is designed by combining a helical tube and a stainless steel tube. The stainless steel tube wraps an outer wall of the helical tube. The sheath core adopts the structure that the helical tube is located at a distal end while the stainless steel tube is located at a proximal end. A notch is provided at a distal end of the stainless steel tube, and the distal end is a closed section for being welded and fixed to the helical tube and sealing the notch. The notch achieves a flexible even transition between the helical tube and the stainless steel tube, ensures the flexibility of the distal end of the sheath core 10 and the rigidity of the proximal end, and ensures that a main body of the sheath core 10 has sufficient pushing property. The sheath core assembly 100 of this embodiment not only solves the problem of difficulty in withdrawing caused by the steps in the welding zone, but also solves the problem of difficulty in pushing and withdrawing caused by the poor compliance of the distal end of the existing sheath core 10.

### Embodiment 2

Referring to FIG. 1 and FIG. 4, as mentioned in the Background Art, the sheath core assembly of the existing delivery device further includes a bifurcation tube 2 for connecting and fixing the tip 1 to the stainless steel tube 4. The structure of the existing bifurcation tube 2 is shown in FIG. 4. The bifurcation tube 2 has a welding zone 2-1 and a fixing zone 2-2. The two functional zones are arranged at different positions of the bifurcation tube 2, resulting in a large overall length L1 of the bifurcation tube. Also, since the bifurcation tube 2 is buried in the tip 1, a length L2 of a high-hardness zone of the tip 1 is large, thus reducing the compliance of the tip 1 and affecting the pushing and withdrawing of the punctured tissue of the sheath core 4 in the blood vessel. In addition, when the bifurcation tube 2 is used for injection molding of the tip 1, the fixing zone 2-2 of the bifurcation tube 2 will be deformed or even broken in an arrow direction due to the injection molding pressure (the pressure direction is as indicated by arrows in FIG. 4), which will reduce the connection strength and seriously lead to the connection failure.

In view of this, this embodiment provides a sheath core assembly, so as to solve the problem of fracture failure.

Referring to FIG. 7 and FIG. 10, this embodiment provides a sheath core assembly 100. The sheath core assembly 100 of this embodiment includes a sheath core 10, a U-shaped anchor sleeved to the sheath core 10 and relatively fixed to the sheath core 10, and a connector 30 sleeved to a distal end of the sheath core 10 and configured to connect the sheath core 10 to a tip 40. The sheath core assembly 100 includes a tip 40 wrapping the distal end of the sheath core 10. The structure of the sheath core 10 in this embodiment may refer to the prior art. For example, the sheath core may be a stainless steel tube. Preferably, a novel sheath core structure may also be provided. A detailed implementation of the novel sheath core structure may refer to the following Embodiment 3. The specific structure of the U-shaped anchor is described in detail with reference to the U-shaped anchor 20 in Embodiment 1 and will not be described herein. In this embodiment, the structure of the connector 30 sleeved to the distal end of the sheath core 10 and configured to connect the sheath core 10 to the tip 40 is mainly described in detail. It should be noted that in addition to the structure of the connector 30 in this embodiment, the structure of other features may refer to the prior art or other embodiments of this application, and the structure of other features is not limited.

As shown in FIG. 7, FIG. 10, and FIG. 11, the connector 30 for connecting the sheath core 10 to the tip 40 in this embodiment is a solid metal member. Exemplarily, the connector is machined from a stainless steel material. The connector 30 is axially provided with an axial hole 31. The connector 30 is sleeved to the distal end of the sheath core 10 and relatively fixed to the sheath core 10 through the axial hole 31. A groove is recessed in an outer peripheral surface of the connector 30, and the connector 30 is relatively fixed to the sheath core 10 and the tip 40 through the groove. With the groove, the thickness of the groove can be reduced, thus realizing the welding between the connector 30 and the sheath core 10 through the groove. In addition, during the injection molding process of the tip 40, glue injection liquid of the tip 40 may enter the groove to realize the connection between the tip 40 and the connector 30, while forming axial and circumferential limits.

Exemplarily, the connector 30 has a polygonal radial cross-section, whereby an outer peripheral face of the connector 30 includes multiple surfaces, and the groove is recessed in the outer peripheral surface of the connector 30. This arrangement further enhances the connection and improves the axial and circumferential limits. The groove may be provided on one surface, grooves are provided on multiple surfaces, or multiple grooves are provided on one surface, which may be specifically set according to the situation. It should also be noted that the radial cross-section of the connector 30 may be of other shapes, and is not limited to a polygon. For example, the radial cross-section of the connector may be round, and the connector 30 may be a cylindrical body with a groove recessed in a surface of the cylindrical body.

As shown in FIG. 11, as an implementation, the connector 30 has a quadrilateral radial cross-section, whereby the outer peripheral face of the connector 30 includes four surfaces. Preferably, for ease of machining, the connector 30 is a cuboid or a cube, where a circumferential surface of the cuboid or the cube is provided with a groove. Exemplarily, each outer peripheral surface of the cuboid or the cube is provided with a first groove 32 penetrating a distal face and a proximal face of the connector 30, so as to form a strip-shaped groove on each outer peripheral surface. The groove bottom of the strip-shaped groove is welded to the sheath core 10 to achieve relative fixation between the connector 30 and the sheath core 10.

As shown in FIG. 12, as another implementation, the connector 30 has a quadrilateral radial cross-section, whereby the outer peripheral face of the connector 30 includes four surfaces. Preferably, for ease of machining, the connector 30 is a cuboid or a cube, where a circumferential surface of the cuboid or the cube is provided with a second groove 33 not penetrating the distal face and the proximal face of the connector 30. Exemplarily, the second groove 33 is an annular groove surrounding an outer peripheral surface of the cuboid or the cube. The groove bottom of the annular groove is welded to the sheath core 10 to achieve relative fixation between the connector 30 and the sheath core 10.

As shown in FIG. 13, as yet another implementation, the connector 30 has a quadrilateral radial cross-section, whereby the outer peripheral face of the connector 30 includes four surfaces. Preferably, for ease of machining, the connector 30 is a cuboid or a cube, where a circumferential surface of the cuboid or the cube is provided with a groove. Exemplarily, each outer peripheral surface of the cuboid or the cube is provided with a first groove 32 penetrating a distal face and a proximal face of the connector 30, so as to form a strip-shaped groove on each outer peripheral surface. At the same time, a circumferential surface of the cuboid or the cube is provided with a second groove 33 not penetrating the distal face and the proximal face of the connector 30. The second groove 33 is provided annularly. The annular groove penetrates through the strip-shaped groove. The groove bottoms of the grooves are welded to the sheath core 10 to achieve relative fixation between the connector 30 and the sheath core 10.

As shown in FIG. 14, as an implementation that the connector 30 has a quadrilateral radial cross-section, a third groove 34 is recessed circumferentially along the outer peripheral face of the connector 30. The third groove 34 does not penetrate the distal face and the proximal face of the connector 30, whereby blocking portions 30b are formed at a proximal side and a distal side of the third groove 34 respectively. The blocking portions 30b have quadrilateral radial cross-sections, and two opposite sides of the quadrilateral are straight lines while the other two opposite sides are arc lines, whereby two opposite faces of the blocking portion 30b are arc faces, and the arc faces of the two blocking portions 30b at the proximal side and the distal side are interlaced in azimuth. The interlaced blocking portions 30b further improve the strength of the circumferential limit and the axial limit.

In this embodiment, the welding zone and the circumferential fixing zone of the connector 30 are designed in the same groove. As shown in FIG. 11 compared to FIG. 4, an overall length L3 of the connector 30 of this embodiment is obviously smaller than a length L4 of the existing bifurcation tube, and the overall length is reduced. As shown in FIG. 7, after being buried in the tip 40, the length L4 of a high-hardness zone of the tip 40 is smaller, and the compliance is better. At the same time, the overall structure of the connector 30 is more stable, and the connector will not be deformed during injection molding of the tip 40 and is more reliably connected to the tip 40. The sheath core assembly 100 of this embodiment solves the problems of difficulty in pushing and withdrawing and fracture failure caused by the existing bifurcation tube. Definitely, when the U-shaped anchor of this embodiment adopts the U-shaped anchor 20 of Embodiment 1, this embodiment solves the problems of difficulty in pushing and withdrawing and fracture failure while further solving the problem of difficulty in withdrawing caused by the steps in the welding zone.

Further, as shown in FIG. 1 and FIG. 6, as mentioned in the Background Art, the existing sheath core assembly includes a sheath core 4. The sheath core 4 is usually a stainless steel tube, which leads to high hardness and poor compliance of a distal end of the sheath core 4. The sheath core cannot bend or recover in time in compliance with the shape of a blood vessel a, and the blood vessel a conflicts with the sheath core 4 to form a resistance zone a1, which will make it difficult to push or withdraw the sheath core.

In view of this, as an optimized scheme, on the basis of solving the problem of fracture failure, this embodiment further solves the problem of difficulty in pushing and withdrawing caused by high hardness of the distal end of the sheath core.

In an optimized scheme, the sheath core assembly of this embodiment includes a sheath core 10 and a U-shaped anchor 20 sleeved to the sheath core 10 and relatively fixed to the sheath core 10. Preferably, the sheath core assembly 100 may further include a tip 40 wrapping the distal end of the sheath core 10. It should be noted that the tip 40 does not constitute a necessary feature of this embodiment to solve the problems presented therein. It should be noted that in addition to the structure of the sheath core 10 and the U-shaped anchor 20 in this embodiment, the structure of other features may refer to the prior art or other embodiments of this application, and the structure of other features is not limited.

Specifically, as shown in FIG. 7, FIG. 10, and FIG. 15, the sheath core 10 of this embodiment includes a first tube body 11 and a second tube body 12 sleeved in the first tube body 11. A distal end of the second tube body 12 extends out of a distal face of the first tube body 11, and the compliance of the second tube body 12 is superior to that of the first tube body 11. The U-shaped anchor 20 is sleeved to the second tube body 12 and relatively fixed to the second tube body 12. In this embodiment, the second tube body 12 having better compliance is sleeved to the first tube body 11 and extends out of the distal end of the first tube body 11, thereby improving the hardness of the distal end of the sheath core 10, improving the compliance of the distal end of the sheath core 10, and solving the problem of difficulty in pushing and withdrawing caused by high hardness of the distal end of the sheath core 10.

Exemplarily, referring to FIG. 15, the first tube body 11 is a cylindrical tube, preferably a stainless steel tube. The second tube body 12 is a helical tube, having a helical structure similar to a spring. The stainless steel tube wraps an outer wall of the helical tube, and a distal end of the helical tube extends out of a distal end of the stainless steel tube. The compliance of the helical tube is superior to that of the stainless steel tube, and the rigidity of the distal end is ensured while the flexibility of the proximal end of the sheath core 10 is ensured, and a main body of the sheath core 10 has sufficient pushing property.

There is a gap between the helical tube and the stainless steel tube because of the sleeve connection between the helical tube and the stainless steel tube, and there is usually a final cleaning process in the manufacturing process, in which cleaning liquid will enter the gap and flush the gap at the same time. In view of this, as shown in FIG. 16 and FIG. 17, the first tube body 11 of this embodiment includes a first section 11a sleeved outside the second tube body 12 and a second section 11b connected to a distal end of the first section 11a. The first section 11a is provided with a notch 11a1. The sleeved stainless steel tube and helical tube can be fully flushed through the notch 11a1, and flushing liquid can easily flow out of the notch after the cleaning process, thus avoiding the cleaning liquid remaining in the gap. At the same time, the hardness of a sleeving section between the first tube body 11 and the second tube body 12 is transitioned to avoid high local hardness, whereby the compliance of the sheath core 10 is better. It should be noted that the distal end of the first section 11a is closed so that the end has a closed section by which the distal ends of the first tube body 11 and the second tube body 12 can be welded.

Preferably, multiple notches 11a1 are provided at intervals along an axial direction of the first section 11a, and the entire sleeving section can be fully cleaned by the multiple notches 11a1 in the axial direction while the hardness of the entire section is transitioned. As an implementation, the adjacent notches 11a1 on the first section 11a are provided at equal intervals from a proximal end of the first section 11a to the distal end thereof. As another implementation, the adjacent notches 11a1 on the first section 11a are provided at gradually reducing intervals from a proximal end of the first section 11a to the distal end thereof. The distance between the proximal and distal notches 11a1 is gradually reduced, whereby the adjacent notches 11a1 are gradually increased from the proximal end to the distal end, and the hardness transition from the proximal end to the distal end is better, and the flexibility towards the distal end is better.

With continued reference to FIG. 16 and FIG. 17, in other embodiments, the notch 11a1 is a helical notch which extends helically along an axial direction of the first section 11a in order to facilitate the cleaning of the gap within the entire sleeving section at the same time as machining. Preferably, as an implementation, the adjacent notches 11a1 on the first section 11a are provided at equal intervals W from a proximal end of the first section 11a to the distal end thereof. As another implementation, the adjacent notches 11a1 on the first section 11a are provided at gradually reducing intervals W from a proximal end of the first section 11a to the distal end thereof, whereby the adjacent notches 11a1 are gradually increased from the proximal end to the distal end, and the transition from the proximal end to the distal end is better, and the flexibility towards the distal end is better.

In the sleeving process, preferably, as shown in FIG. 16, a length L5 between the distal and the proximal notches 11a1 on the first section 11a of this embodiment is 5-200 mm. This length orientation ensures as much as possible the compliance of the transition section and the rigidity of other sections of the sheath core 10, whereby the main body of the sheath core 10 has sufficient pushing property.

In this embodiment, the sheath core 10 is designed by combining a helical tube and a stainless steel tube. The stainless steel tube wraps an outer wall of the helical tube. The sheath core adopts the structure that the helical tube is located at a distal end while the stainless steel tube is located at a proximal end. A notch is provided at a distal end of the stainless steel tube, and the distal end is a closed section for being welded and fixed to the helical tube and sealing the notch. The notch achieves a flexible even transition between the helical tube and the stainless steel tube, ensures the flexibility of the distal end of the sheath core 10 and the rigidity of the proximal end, and ensures that a main body of the sheath core 10 has sufficient pushing property. The sheath core assembly 100 of this embodiment not only solves the problem of fracture failure, but also solves the problem of difficulty in pushing and withdrawing caused by the poor compliance of the distal end of the existing sheath core 10.

### Embodiment 3

As shown in FIG. 1 and FIG. 6, as mentioned in the Background Art, the existing sheath core assembly includes a sheath core 4. The sheath core 4 is usually a stainless steel tube, which leads to high hardness and poor compliance of a distal end of the sheath core 4. The sheath core cannot bend or recover in time in compliance with the shape of a blood vessel a, and the blood vessel a conflicts with the sheath core 4 to form a resistance zone a1, which will make it difficult to push or withdraw the sheath core.

In view of this, this embodiment provides a novel sheath core assembly, so as to solve the problem of difficulty in pushing and withdrawing caused by high hardness of the distal end of the sheath core.

Referring to FIG. 7 and FIG. 10, the sheath core assembly 100 of this embodiment includes a sheath core 10 and a U-shaped anchor sleeved to the sheath core 10 and relatively fixed to the sheath core 10. In addition, the sheath core assembly 100 may further include a tip 40 wrapping a distal end of the sheath core 10. The specific structure of the U-shaped anchor in this embodiment refers to the U-shaped anchor 20 in Embodiment 1 and will not be described herein. This embodiment mainly describes in detail the structure of the sheath core in the sheath core assembly 100. It should be noted that in addition to the structure of the sheath core 10 in this embodiment, the structure of other features may refer to the prior art or other embodiments of this application, and the structure of other features is not limited.

Specifically, as shown in FIG. 7, FIG. 10, and FIG. 15, the sheath core 10 of this embodiment includes a first tube body 11 and a second tube body 12 sleeved in the first tube body 11. A distal end of the second tube body 12 extends out of a distal face of the first tube body 11, and the compliance of the second tube body 12 is superior to that of the first tube body 11. The U-shaped anchor 20 is sleeved to the second tube body 12 and relatively fixed to the second tube body 12.

In this embodiment, the second tube body 12 having better compliance is sleeved to the first tube body 11 and extends out of the distal end of the first tube body 11, thereby improving the hardness of the distal end of the sheath core 10, improving the compliance of the distal end of the sheath core 10, and solving the problem of difficulty in pushing and withdrawing caused by high hardness of the distal end of the sheath core 10.

Exemplarily, referring to FIG. 15, the first tube body 11 is a cylindrical tube, preferably a stainless steel tube. The second tube body 12 is a helical tube, having a helical structure similar to a spring. The stainless steel tube wraps an outer wall of the helical tube, and a distal end of the helical tube extends out of a distal end of the stainless steel tube. The compliance of the helical tube is superior to that of the stainless steel tube, and the rigidity of the distal end is ensured while the flexibility of the proximal end of the sheath core 10 is ensured, and a main body of the sheath core 10 has sufficient pushing property.

There is a gap between the helical tube and the stainless steel tube because of the sleeve connection between the helical tube and the stainless steel tube, and there is usually a final cleaning process in the manufacturing process, in which cleaning liquid will enter the gap and flush the gap at the same time. In view of this, as shown in FIG. 16 and FIG. 17, the first tube body 11 of this embodiment includes a first section 11a sleeved outside the second tube body 12 and a second section 11b connected to a distal end of the first section 11a. The first section 11a is provided with a notch 11a1. The sleeved stainless steel tube and helical tube can be fully flushed through the notch 11a1, and flushing liquid can easily flow out of the notch after the cleaning process, thus avoiding the cleaning liquid remaining in the gap. At the same time, the hardness of a sleeving section between the first tube body 11 and the second tube body 12 is transitioned to avoid high local hardness, whereby the compliance of the sheath core 10 is better. It should be noted that the distal end of the first section 11a is closed so that the end has a closed section by which the distal ends of the first tube body 11 and the second tube body 12 can be welded.

Preferably, multiple notches 11a1 are provided at intervals along an axial direction of the first section 11a, and the entire sleeving section can be fully cleaned by the multiple notches 11a1 in the axial direction while the hardness of the entire section is transitioned. As an implementation, the adjacent notches 11a1 on the first section 11a are provided at equal intervals from a proximal end of the first section 11a to the distal end thereof. As another implementation, the adjacent notches 11a1 on the first section 11a are provided at gradually reducing intervals from a proximal end of the first section 11a to the distal end thereof. The distance between the proximal and distal notches 11a1 is gradually reduced, whereby the adjacent notches 11a1 are gradually increased from the proximal end to the distal end, and the hardness transition from the proximal end to the distal end is better, and the flexibility towards the distal end is better.

With continued reference to FIG. 16 and FIG. 17, in other embodiments, the notch 11a1 is a helical notch which extends helically along an axial direction of the first section 11a in order to facilitate the cleaning of the gap within the entire sleeving section at the same time as machining. Preferably, as an implementation, the adjacent notches 11a1 on the first section 11a are provided at equal intervals W from a proximal end of the first section 11a to the distal end thereof. As another implementation, the adjacent notches 11a1 on the first section 11a are provided at gradually reducing intervals W from a proximal end of the first section 11a to the distal end thereof, whereby the adjacent notches 11a1 are gradually increased from the proximal end to the distal end, and the transition from the proximal end to the distal end is better, and the flexibility towards the distal end is better.

In the sleeving process, preferably, as shown in FIG. 16, a length L5 between the distal and the proximal notches 11a1 on the first section 11a of this embodiment is 5-200 mm. This length orientation ensures as much as possible the compliance of the transition section and the rigidity of other sections of the sheath core 10, whereby the main body of the sheath core 10 has sufficient pushing property.

In this embodiment, the sheath core 10 is designed by combining a helical tube and a stainless steel tube. The stainless steel tube wraps an outer wall of the helical tube. The sheath core adopts the structure that the helical tube is located at a distal end while the stainless steel tube is located at a proximal end. A notch is provided at a distal end of the stainless steel tube, and the distal end is a closed section for being welded and fixed to the helical tube and sealing the notch. The notch achieves a flexible even transition between the helical tube and the stainless steel tube, ensures the flexibility of the distal end of the sheath core 10 and the rigidity of the proximal end, and ensures that a main body of the sheath core 10 has sufficient pushing property. The sheath core assembly 100 of this embodiment solves the problem of difficulty in pushing and withdrawing caused by the poor compliance of the distal end of the existing sheath core 10.

### Embodiment 4

Referring to FIG. 7, FIG. 10, and FIG. 15, on the basis of Embodiments 1 and 2, this embodiment further provides a sheath core assembly 100. The sheath core assembly 100 of this embodiment includes a sheath core 10, a U-shaped anchor 20 sleeved to the sheath core 10 and relatively fixed to the sheath core 10, a connector 30 sleeved to a distal end of the sheath core 10 and configured to connect the sheath core 10 to a tip 40, and the tip 40 wrapping the distal end of the sheath core 10. The specific structure of the U-shaped anchor 20 in this embodiment refers to Embodiment 1 and will not be described herein. The specific structure of the connector 30 in this embodiment refers to Embodiment 2 and will not be described herein. The specific structure of the sheath core 10 in this embodiment refers to Embodiment 3 and will not be described herein.

In this embodiment, the sheath core 10 is designed by combining a helical tube and a stainless steel tube. The stainless steel tube wraps an outer wall of the helical tube. The sheath core adopts the structure that the helical tube is located at a distal end while the stainless steel tube is located at a proximal end. A notch is provided at a distal end of the stainless steel tube, and the distal end is a closed section for being welded and fixed to the helical tube and sealing the notch. The notch achieves a flexible even transition between the helical tube and the stainless steel tube, ensures the flexibility of the distal end of the sheath core 10 and the rigidity of the proximal end, and ensures that a main body of the sheath core 10 has sufficient pushing property. The sheath core assembly 100 of this embodiment not only solves the problem of difficulty in withdrawing caused by the steps in the welding zone, but also solves the problems of difficulty in pushing and withdrawing and fracture failure caused by the existing bifurcation tube and the problem of difficulty in pushing and withdrawing caused by the poor compliance of the distal end of the existing sheath core 10.

### Embodiment 5

This embodiment provides a delivery instrument. The delivery instrument includes any sheath core assembly 100 in Embodiment 1 to Embodiment 4.

The present invention provides a sheath core assembly and a delivery instrument including the sheath core assembly. An outer periphery of an anchor body is arranged as an arc-shaped transition face, and a welding groove is recessed in the arc-shaped transition face, whereby a distal end of the anchor body is stepless, thus solving the problem of difficulty in pushing and withdrawing due to steps in a welding zone, and greatly improving the smoothness of withdrawal of a delivery device in blood vessels, especially curved blood vessels. In addition, built-in welding spots avoid damage on the blood vessels by surface unevenness caused by welding, thus further improving the smoothness.

The present invention provides a sheath core assembly and a delivery instrument including the sheath core assembly. By designing a welding zone and a circumferential fixing zone of a connector in the same groove, an overall length of the connector is obviously smaller than the length of the existing bifurcation tube. By reducing the overall length, after the connector is buried in a tip, the length of a high-hardness zone of the tip is smaller, and the compliance is better. At the same time, the overall structure of the connector is more stable, and the connector will not be deformed during injection molding of the tip and is more reliably connected to the tip, thus solving the problems of difficulty in pushing and withdrawing and the fracture failure caused by the existing bifurcation tube.

The present invention provides a sheath core assembly and a delivery instrument including the sheath core assembly. A sheath core 10 is designed by combining a helical tube and a stainless steel tube. The stainless steel tube wraps an outer wall of the helical tube. The sheath core adopts the structure that the helical tube is located at a distal end while the stainless steel tube is located at a proximal end. A notch is provided at a distal end of the stainless steel tube, and the distal end is a closed section for being welded and fixed to the helical tube and sealing the notch. The notch achieves a flexible even transition between the helical tube and the stainless steel tube, ensures the flexibility of the distal end of the sheath core 10 and the rigidity of the proximal end, ensures that a main body of the sheath core 10 has sufficient pushing property, and solves the problem of difficulty in pushing and withdrawing caused by the poor compliance of the distal end of the existing sheath core.

The various technical features of the above embodiments may be combined randomly. In order to simplify the description, all possible combinations of the various technical features in the above-mentioned embodiments are not described. However, as long as the combinations of these technical features do not contradict, the combinations should be considered to be the scope of the description.

The above-mentioned embodiments express only a few implementations of the present invention, which are described in greater detail but are not to be construed as limiting the scope of the present invention. It will be appreciated by those of ordinary skill in the art that numerous variations and modifications may be made to the present invention, as long as the resulting embodiment falls within the scope of the appended claims.

## Claims

1. A sheath core assembly (100), comprising a sheath core (10) and a U-shaped anchor (20), wherein the U-shaped anchor (20) comprises an anchor body (21) and a stent fixing rod (22); the anchor body (21) is sleeved to the sheath core (10) and relatively fixed to the sheath core (10); the anchor body comprises a distal face (21a) and a proximal face (21b) which are opposite to each other, and an outer peripheral face (21c) surrounded between the distal face (21a) and the proximal face (21b); the distal face (21a) is larger than the proximal face (21b), and edge profiles of the distal face (21a) and the proximal face (21b) are arc-shaped; the outer peripheral face (21c) is an arc face having a smooth transition from the distal face (21a) to the proximal face (21b); and a proximal end of the stent fixing rod (22) is fixedly connected to the distal face (21a) of the anchor body (21), wherein
the sheath core assembly (100) further comprising a tip (40) wrapping a distal end of the sheath core (10) and a connector (30) sleeved to a distal end of the sheath core (10) and configured to connect the sheath core (10) to the tip (40), wherein a groove is recessed in an outer peripheral surface of the connector (30), and the connector (30) is relatively fixed to the sheath core (10) and the tip (40) through the groove.

2. The sheath core assembly (100) according to claim 1, wherein the distal face (21a) and the proximal face (21b) have the same shape.

3. The sheath core assembly (100) according to claim 2, wherein both the distal face (21a) and the proximal face (21b) of the anchor body (21) are round, whereby the anchor body (21) is approximately conical; or wherein both the distal face (21a) and the proximal face (21b) of the anchor body (21) are elliptic, whereby the anchor body (21) is approximately elliptically conical.

4. The sheath core assembly (100) according to claim 3, wherein an accommodating groove (23) penetrating the distal face (21a) is recessed in the outer peripheral face (21c) near a distal end of the anchor body (21), and the accommodating groove (23) is provided near the stent fixing rod (22), whereby a connecting arm of a stent hooked on the stent fixing rod (22) is placed in the accommodating groove (23).

5. The sheath core assembly (100) according to claim 1, wherein a welding groove (24) is recessed in the outer peripheral face (21c) near the proximal end of the anchor body (21), wherein multiple welding grooves (24) are provided at intervals circumferentially along the outer peripheral face (21c) of the anchor body (21).

6. The sheath core assembly (100) according to claim 1, wherein the anchor body (21) is axially provided with two symmetrical pulling wire holes (25), and the pulling wire holes (25) are opposite to exhaust grooves (41) on the tip (40) in the sheath core assembly (100).

7. The sheath core assembly (100) according to claim 1, wherein the connector (30) has a polygonal radial cross-section, whereby an outer peripheral face of the connector (30) comprises multiple surfaces, and the groove is recessed in the outer peripheral surface of the connector (30).

8. The sheath core assembly (100) according to claim 7, wherein the connector (30) has a quadrilateral radial cross-section, whereby the outer peripheral face of the connector (30) comprises four surfaces, and the groove comprises a first groove (32) provided on the surface and penetrating a distal face and a proximal face of the connector (30) and/or comprises a second groove (33) recessed circumferentially along the outer peripheral face of the connector (30).

9. The sheath core assembly (100) according to claim 1, wherein the groove comprises a third groove (34) recessed circumferentially along the outer peripheral face of the connector (30), blocking portions (30b) are formed at a proximal side and a distal side of the third groove (34) respectively and have quadrilateral radial cross-sections, and two opposite sides of the quadrilateral are straight lines while the other two opposite sides are arc lines, whereby two opposite faces of the blocking portion (30b) are arc faces, and the arc faces of the two blocking portions (30b) at the proximal side and the distal side are interlaced in azimuth.

10. The sheath core assembly (100) according to any one of claims 1 to 6, wherein the sheath core (10) comprises a first tube body (11) and a second tube body (12) sleeved in the first tube body (11), a distal end of the second tube body (12) extends out of a distal face of the first tube body (11), and the compliance of the second tube body (12) is superior to that of the first tube body (11).

11. The sheath core assembly (100) according to claim 10, wherein the first tube body (11) is a cylindrical tube, and the second tube body (12) is a helical tube.

12. The sheath core assembly (100) according to claim 10, wherein the first tube body (11) comprises a first section (11a) sleeved outside the second tube body (12) and a second section (11b) connected to a distal end of the first section (11), and the first section (11a) is provided with a notch (11a1), wherein multiple notches (11a1) are provided at intervals along an axial direction of the first section (11a),
wherein the adjacent notches (11a1) on the first section (11) are provided at equal intervals from a proximal end of the first section (11) to the distal end thereof,
or wherein the adjacent notches (11a1) on the first section (11) are provided at gradually reducing intervals from a proximal end of the first section (11) to the distal end thereof.

13. The sheath core assembly (100) according to claim 12, wherein the notch (11a1) is a helical notch which extends helically along an axial direction of the first section (11a).

14. The sheath core assembly (100) according to claim 12 or 13, wherein a length between the nearest and farthest notches (11a1) on the first section (11a) is 5-200 mm.

## Patentansprüche

1. Hüllenkernanordnung (100), einen Hüllenkern (10) und einen U-förmigen Anker (20) umfassend, wobei der U-förmige Anker (20) einen Ankerkörper (21) und einen Stent-Befestigungsstab (22) umfasst; der Ankerkörper (21) auf dem Hüllenkern (10) sitzt und relativ zum Hüllenkern (10) befestigt ist; der Ankerkörper eine distale Fläche (21a) und eine proximale Fläche (21b), die einander gegenüberliegen, und eine äußere Umfangsfläche (21c), die zwischen der distalen Fläche (21a) und der proximalen Fläche (21b) umläuft, umfasst; die distale Fläche (21a) größer als die proximale Fläche (21b) ist und die Kantenprofile der distalen Fläche (21a) und der proximalen Fläche (21b) bogenförmig sind; die äußere Umfangsfläche (21c) eine Bogenfläche ist, die einen glatten Übergang von der distalen Fläche (21a) zur proximalen Fläche (21b) darstellt; und ein proximales Ende des Stent-Befestigungsstabs (22) fest mit der distalen Fläche (21a) des Ankerkörpers (21) verbunden ist, wobei
die Hüllenkernanordnung (100) ferner eine Spitze (40), die ein distales Ende des Hüllenkerns (10) umschließt, und einen Verbinder (30), der an einem distalen Ende des Hüllenkerns (10) sitzt und dafür konfiguriert ist, den Hüllenkern (10) mit der Spitze (40) zu verbinden, umfasst, wobei eine Vertiefung in einer äußeren Umfangsfläche des Verbinders (30) vertieft ausgebildet ist, und der Verbinder (30) relativ zu dem Hüllenkern (10) und der Spitze (40) durch die Vertiefung befestigt ist.

2. Hüllenkernanordnung (100) nach Anspruch 1, wobei die distale Fläche (21a) und die proximale Fläche (21b) dieselbe Form aufweisen.

3. Hüllenkernanordnung (100) nach Anspruch 2, wobei sowohl die distale Fläche (21a) als auch die proximale Fläche (21b) des Ankerkörpers (21) rund sind, wodurch der Ankerkörper (21) annähernd konisch ist; oder wobei sowohl die distale Fläche (21a) als auch die proximale Fläche (21b) des Ankerkörpers (21) elliptisch sind, wodurch der Ankerkörper (21) annähernd elliptisch-konisch ist.

4. Hüllenkernanordnung (100) nach Anspruch 3, wobei eine Aufnahmevertiefung (23), die die distale Fläche (21a) durchdringt, in der äußeren Umfangsfläche (21c) nahe einem distalen Ende des Ankerkörpers (21) vertieft ausgebildet ist, und die Aufnahmevertiefung (23) nahe dem Stent-Befestigungsstab (22) vorgesehen ist, wodurch ein Verbindungsarm eines an dem Stent-Befestigungsstab (22) eingehakten Stents in die Aufnahmevertiefung (23) eingesetzt wird.

5. Hüllenkernanordnung (100) nach Anspruch 1, wobei eine Schweißvertiefung (24) in der äußeren Umfangsfläche (21c) nahe dem proximalen Ende des Ankerkörpers (21) vertieft ausgebildet ist, wobei eine Vielzahl von Schweißvertiefungen (24) in Abständen umlaufend um die äußere Umfangsfläche (21c) des Ankerkörpers (21) vorgesehen sind.

6. Hüllenkernanordnung (100) nach Anspruch 1, wobei der Ankerkörper (21) axial mit zwei symmetrischen Zugdrahtöffnungen (25) vorgesehen ist, und die Zugdrahtöffnungen (25) sich gegenüber Austrittsvertiefungen (41) an der Spitze (40) in der Hüllenkernanordnung (100) befinden.

7. Hüllenkernanordnung (100) nach Anspruch 1, wobei der Verbinder (30) einen polygonalen radialen Querschnitt aufweist, wodurch eine äußere Umfangsfläche des Verbinders (30) mehrere Flächen umfasst, und die Vertiefung in der äußeren Umfangsfläche des Verbinders (30) vertieft ausgebildet ist.

8. Hüllenkernanordnung (100) nach Anspruch 7, wobei der Verbinder (30) einen viereckigen radialen Querschnitt aufweist, wodurch die äußere Umfangsfläche des Verbinders (30) vier Flächen umfasst, und die Vertiefung eine erste Vertiefung (32), die an der Fläche vorgesehen ist und eine distale Fläche und eine proximale Fläche des Verbinders (30) durchdringt, umfasst, und/oder eine zweite Vertiefung (33), die umlaufend um die äußere Umfangsfläche des Verbinders (30) vertieft ausgebildet ist, umfasst.

9. Hüllenkernanordnung (100) nach Anspruch 1, wobei die Vertiefung eine dritte Vertiefung (34) umfasst, die umlaufend um die äußere Umfangsfläche des Verbinders (30) vertieft ausgebildet ist, Sperrabschnitte (30b) an einer proximalen Seite bzw. einer distalen Seite der dritten Vertiefung (34) ausgebildet sind und viereckige radiale Querschnitte aufweisen, und zwei gegenüberliegende Seiten des Vierecks gerade Linien sind, während die anderen beiden gegenüberliegenden Seiten bogenförmige Linien sind, wodurch zwei gegenüberliegende Flächen des Sperrabschnitts (30b) bogenförmige Flächen sind und die bogenförmigen Flächen der zwei Sperrabschnitte (30b) an der proximalen Seite und an der distalen Seite azimutal verschränkt sind.

10. Hüllenkernanordnung (100) nach einem der Ansprüche 1 bis 6, wobei der Hüllenkern (10) einen ersten Rohrkörper (11) und einen zweiten Rohrkörper (12), der im ersten Rohrkörper (11) sitzt, umfasst, ein distales Ende des zweiten Rohrkörpers (12) sich aus einer distalen Fläche des ersten Rohrkörpers (11) erstreckt und die Nachgiebigkeit des zweiten Rohrkörpers (12) größer ist als die des ersten Rohrkörpers (11).

11. Hüllenkernanordnung (100) nach Anspruch 10, wobei der erste Rohrkörper (11) ein zylindrisches Rohr ist und der zweite Rohrkörper (12) ein wendelförmiges Rohr ist.

12. Hüllenkernanordnung (100) nach Anspruch 10, wobei der erste Rohrkörper (11) einen ersten Abschnitt (11a) umfasst, der außerhalb des zweiten Rohrkörpers (12) sitzt, und einen zweiten Abschnitt (11b), der mit einem distalen Ende des ersten Abschnitts (11) verbunden ist, und der erste Abschnitt (11a) eine Kerbe (11a1) aufweist, wobei eine Vielzahl von Kerben (11a1) in Abständen entlang einer axialen Richtung des ersten Abschnitts (11a) vorgesehen sind,
wobei die benachbarten Kerben (11a1) an dem ersten Abschnitt (11) in gleichen Abständen von einem proximalen Ende des ersten Abschnitts (11) zu dessen distalen Ende vorgesehen sind,
oder wobei die benachbarten Kerben (11a1) an dem ersten Abschnitt (11) in allmählich abnehmenden Abständen von einem proximalen Ende des ersten Abschnitts (11) zu dessen distalen Ende vorgesehen sind.

13. Hüllenkernanordnung (100) nach Anspruch 12, wobei die Kerbe (11a1) eine wendelförmige Kerbe ist, die sich wendelförmig gewunden entlang einer axialen Richtung des ersten Abschnitts (11a) erstreckt.

14. Hüllenkernanordnung (100) nach Anspruch 12 oder 13, wobei eine Länge zwischen der nächstgelegenen und der am weitesten entfernten Kerbe (11a1) an dem ersten Abschnitt (11a) 5-200 mm beträgt.

## Revendications

1. Ensemble âme de gaine (100), comprenant une âme de gaine (10) et une ancre en forme de U (20), ladite ancre en forme de U (20) comprenant un corps d'ancre (21) et une tige de fixation de stent (22) ; ledit corps d'ancre (21) manchonnant l'âme de gaine (10) et étant relativement fixé à l'âme de gaine (10) ; ledit corps d'ancre comprenant une face distale (21a) et une face proximale (21b) qui sont opposées l'une à l'autre, et une face périphérique externe (21c) entourée entre la face distale (21a) et la face proximale (21b) ; ladite face distale (21a) étant plus grande que la face proximale (21b), et lesdits profils de bord de la face distale (21a) et de la face proximale (21b) étant en forme d'arc ; ladite face périphérique externe (21c) étant une face en arc possédant une transition douce de la face distale (21a) à la face proximale (21b) ; et une extrémité proximale de la tige de fixation de stent (22) étant reliée de manière fixe à la face distale (21a) du corps d'ancre (21),
ledit ensemble âme de gaine (100) comprenant en outre une pointe (40) enveloppant une extrémité distale de l'âme de gaine (10) et un connecteur (30) manchonnant une extrémité distale de l'âme de gaine (10) et conçu pour relier l'âme de gaine (10) à la pointe (40), une rainure étant évidée dans une surface périphérique externe du connecteur (30), et ledit connecteur (30) étant relativement fixé à l'âme de gaine (10) et à la pointe (40) à travers la rainure.

2. Ensemble âme de gaine (100) selon la revendication 1, ladite face distale (21a) et ladite face proximale (21b) présentant la même forme.

3. Ensemble âme de gaine (100) selon la revendication 2, à la fois ladite face distale (21a) et ladite face proximale (21b) du corps d'ancre (21) étant rondes, moyennant quoi le corps d'ancre (21) est approximativement conique ; ou à la fois ladite face distale (21a) et ladite face proximale (21b) du corps d'ancre (21) étant elliptiques, moyennant quoi le corps d'ancre (21) est de manière approximative elliptiquement conique.

4. Ensemble âme de gaine (100) selon la revendication 3, une rainure de réception (23) pénétrant la face distale (21a) étant évidée dans la face périphérique externe (21c) à proximité d'une extrémité distale du corps d'ancre (21), et ladite rainure de réception (23) étant prévue à proximité de la tige de fixation de stent (22), moyennant quoi un bras de liaison d'un stent accroché à la tige de fixation de stent (22) étant placé dans la rainure de réception (23).

5. Ensemble âme de gaine (100) selon la revendication 1, une rainure de soudage (24) étant évidée dans la face périphérique externe (21c) à proximité de l'extrémité proximale du corps d'ancre (21), de multiples rainures de soudage (24) étant prévues à intervalles circonférentiellement le long de la face périphérique externe (21c) du corps d'ancre (21).

6. Ensemble âme de gaine (100) selon la revendication 1, ledit corps d'ancre (21) étant pourvu axialement de deux trous de fil de traction symétriques (25), et lesdits trous de fil de traction (25) étant opposés à des rainures d'évacuation (41) sur la pointe (40) dans l'ensemble âme de gaine (100).

7. Ensemble âme de gaine (100) selon la revendication 1, ledit connecteur (30) possédant une section transversale radiale polygonale, une face périphérique externe dudit connecteur (30) comprenant de multiples surfaces et ladite rainure étant évidée dans ladite surface périphérique externe du connecteur (30).

8. Ensemble âme de gaine (100) selon la revendication 7, ledit connecteur (30) possédant une section transversale radiale quadrilatérale, moyennant quoi la face périphérique externe du connecteur (30) comprend quatre surfaces et ladite rainure comprenant une première rainure (32) prévue sur la surface et pénétrant une face distale et une face proximale du connecteur (30) et/ou comprenant une deuxième rainure (33) évidée circonférentiellement le long de la face périphérique externe du connecteur (30).

9. Ensemble âme de gaine (100) selon la revendication 1, ladite rainure comprenant une troisième rainure (34) évidée circonférentiellement le long de la face périphérique externe du connecteur (30), des parties de blocage (30b) étant formées respectivement au niveau d'un côté proximal et d'un côté distal de la troisième rainure (34) et possédant des sections transversales radiales quadrilatérales, et deux côtés opposés du quadrilatère étant des lignes droites pendant que les deux autres côtés opposés sont des lignes d'arc, moyennant quoi deux faces opposées de la partie de blocage (30b) sont des faces d'arc, et les faces d'arc des deux parties de blocage (30b) au niveau du côté proximal et du côté distal sont entrelacées en azimut.

10. Ensemble âme de gaine (100) selon l'une quelconque des revendications 1 à 6, ladite âme de gaine (10) comprenant un premier corps de tube (11) et un second corps de tube (12) manchonné dans le premier corps de tube (11), une extrémité distale du second corps de tube (12) s'étendant hors d'une face distale du premier corps de tube (11), et ladite souplesse du second corps de tube (12) étant supérieure à celle du premier corps de tube (11).

11. Ensemble âme de gaine (100) selon la revendication 10, ledit premier corps de tube (11) étant un tube cylindrique et ledit second corps de tube (12) étant un tube hélicoïdal.

12. Ensemble âme de gaine (100) selon la revendication 10, ledit premier corps de tube (11) comprenant une première section (11a) manchonnée à l'extérieur du second corps de tube (12) et une seconde section (11b) reliée à une extrémité distale de la première section (11), et ladite première section (11a) étant pourvue d'une entaille (11a1), de multiples entailles (11a1) étant prévues à intervalles le long d'une direction axiale de la première section (11a),
lesdites entailles adjacentes (11a1) sur la première section (11) étant disposées à intervalles égaux à partir d'une extrémité proximale de la première section (11) jusqu'à l'extrémité distale de celle-ci,
ou lesdites entailles adjacentes (11a1) sur la première section (11) étant disposées à des intervalles progressivement réduits à partir d'une extrémité proximale de la première section (11) jusqu'à l'extrémité distale de celle-ci.

13. Ensemble âme de gaine (100) selon la revendication 12, ladite entaille (11a1) étant une entaille hélicoïdale qui s'étend de manière hélicoïdale le long d'une direction axiale de ladite première section (11a).

14. Ensemble âme de gaine (100) selon la revendication 12 ou 13, une longueur entre les entailles (11a1) les plus proches et les plus éloignées sur ladite première section (11a) étant 5 à 200 mm.
